# EUROPEAN PATENT APPLICATION

(11) **EP 1 393 696 A1**
(43) Date of publication of application: **03.03.2004**
(21) Application number: 03255220.0
(22) Date of filing: 22.08.2003
(51) Int. Cl.: A61F 2/42, A61B 17/16, A61B 17/17

(54) **Ankle fusion guide and method**

(30) Priority: 26.08.2002 US 226770
(71) Applicant: DePuy Products, Inc., Warsaw, IN 46581 (US)
(72) Inventor: Oberst, Rebecca D., FT Wayne Indiana (US); Conti, Stephen F., Sewickley Philadelphia (US); Maroney, Brian J., Ft Wayne Indiana (US); Long, Jack F., Warsaw Indiana (US)
(74) Representative: Alton, Andrew

(57) **Abstract**

A kit (10) for removal of bone (12) from the talus (14) and the tibia (16) of a patient (20) for performing ankle fusion is provided. The kit (10) includes a guide (22) for cooperation with at least one of the tibia (16) and the talus (14) and an instrument (24) constrainable by the guide (22) for removal of the bone (12).

## Description

The present invention relates generally to the field of orthopaedics, and more particularly, to an implant for use in arthroplasty.

Osteoarthritis and rheumatoid arthritis are common afflictions of the joints of the human body. The ankle is one of the many joints which may be susceptible to osteoarthritis. Arthrodesis has been an accepted treatment for painful osteoarthritis and rheumatoid arthritis of the ankle and the subtailor joints for many years. In the most common of arthrodesis, the talus, tibia and calcaneus are fused together. Such a procedure is commonly known as an ankle fusion. Another less common treatment for arthritis of the ankle is total ankle arthroplasty. Total ankle arthroplasty can be described in greater detail in U.S. Patent 5,326,365 to Alvine, hereby incorporated by reference in its entirety. A portion of the ankle fusion procedure is to resect the distal tibia and the proximal talus. The resected surface of the distal tibia and the proximal talus are then fused together.
The tibia and talus may be fused together using any of a combination of bone plates, bone screws, and intermedullary nails. To perform the tibia and talus resections, the ankle joint is distracted approximately one centimeter. While in this distracted condition, the tibia and talus are resected. The joint is then relaxed and then the tibia and talus are fused.

The resection of the tibia and talus are typically performed utilizing a saw blade that is held in the surgeon's hand and the resection is performed free hand. The free-hand resection of the tibia and talus has several problems. One problem with the current free-hand method of resection is the danger of over resection of the joint surfaces. If too great a resection is performed, the ankle joint height is compromised. The patient then may have a resected leg length that is unacceptably shorter than the unfused leg length. Another problem with the present free- hand method of resecting the tibia and talus is that fore and hind foot alignment may be inaccurate. Alignment is very important because a fused ankle has only a limited degree of flexion. Excessive dorsal flexion or plantar flexion may cause gait problems or patient pain.

An additional problem with the use of a free hand tibia and talus resection is that the cuts may not be straight. If the cuts are not straight or are angled in or out, problems may occur with fusion. If the cuts are not straight, the contact surface between the tibia and talus may be minimal causing excessive minimal contact between the tibia and talus lengthening the healing time and jeopardizing the successfulness of the fusion. Alternatively, if the surfaces are not straight, the tibia and talus may not be properly oriented to each other causing dorsal flexion or planar flexion which may cause gait problems or pain.

Currently, if a patient is considered as a potential total ankle arthroplasty candidate and such surgery is planned, the suitability of such a patient as a candidate cannot be totally determined until the surgery has begun. It will not be until an actual condition of the patient's tibia, talus, and associated soft tissue is observed after the incisions are performed by the surgeon that it can be determined whether the patient is truly a candidate for a total ankle arthroplasty. If the patient's tibia, talus, or soft tissue is sufficiently diseased, the only available remedy for the patient may be ankle fusion. In such a situation, the patient will be required to have an ankle fusion performed utilizing the above mentioned freehanded resection of the tibia and talus.

According to the present invention, an ankle fusion guide is provided as a cutting guide for a tibial and talar resection during an ankle fusion procedure. The guide includes two guiding slots, one each for the tibia and talus cut. The guide may also include a series of pinholes to allow for stabilization of the guide during resection. The guide may be adapted to fit into a total ankle arthroplasty alignment jig, which would align the cutting guide to the neutral position using the tibia. Utilizing the ankle fusion guide in the total ankle arthoplasty jig would permit the surgeon to revert to an ankle fusion procedure easily and quickly when an anticipated total ankle arthroplasty may not be performed due to the deteriorated condition of the patient's bones or soft tissue.

The top of the fusion guide may be designed with a T- shaped top that is even with the anterior face of the guide such that the posterior flush face of the guide is setting against the bone. This configuration eliminates toggling of the saw blade during the resection.

When the ankle fusion guide is attached to the total ankle alignment jig, a neutral alignment with the tibia may be provided.

The ankle fusion guide provides a straight cutting edge for the saw blade ensuring a flat, level cut which makes the fusion easier. The guide may have a T shape top which may be set even to the anterior face of the guide so that the posterior face can sit flush to the bone when attached to the alignment jig. This configuration provides more accuracy during cutting. The ankle fusion guide may include a T shaped top, which is narrow at the base, thereby providing a smaller object to place into the wound site, which permits the surgeon to have a better view of the tibia and the talus.

The ankle fusion cutting guide may also include pinholes, for example, four pin holes. The two middle pin holes may be set straight into the tibia while the other two pin holes, one medial and one lateral, are set into the tibia at an angle. The use of the pinholes assures the stability of the guide during resection.

When utilizing the four pinholes, the middle two holes may be an equal distance from the midline of the device and may be drilled straight through at a 90 degree angle with respect to the device. When utilizing the pinholes, the outer two holes may be placed medially and lateral to the middle holes and may be angled inwardly at an angle, for example, 15 degrees. All four holes may have uniform diameter so that a common drill and pin may be used.

To provide a guide for a resecting saw, the ankle fusion cutting guide may include two slots on the distal portion of the base. These slots may run almost entirely the width of the guide. The slots may be close together and the tibial slot may have starting holes, one being medial and one being lateral. The starting holes guide the drill needed to start the resection and insure a smooth round edge free of stress risers. The talar cutting slot may not need a starting hole. A small hole may be located on one side of the guide and may be used to accommodate a wire for making a wire electronic discharge machine (EDM machine) with the slot. Both cutting slots are preferably thick enough for use with a common, standard saw blade.

According to one embodiment of the present invention, a kit for removal of bone from the talus and the tibia of a patient for performing ankle fusion is provided. The kit includes a guide for cooperation with at least one of the tibia and the talus and an instrument constrainable by the guide for removal of the bone.

According to another embodiment of the present invention, a guide for guiding an instrument for the removal of bone from a first bone and a second bone of a patient for performing joint fusion between the first bone and the second bone is provided. The guide includes a body and a feature operably associated with the body for constraining the instrument.

The feature can comprises a first constraint for guiding a tool for performing the first bone resection and a second constraint for guiding the tool for performing the second bone resection. At least one of said first constraint and said second constraint can be defined by an opening in said body. The opening can be defined by a slot.

Said body can further define a hole therethrough.

Said body can define a first through slot, a second through slot spaced from and parallel to the first slot, and a plurality of circular though holes.

According to yet another embodiment of the present invention, a jig for fixedly positioning a guide for guiding an instrument for the removal of bone from the talus and the tibia of a patient for performing ankle fusion is provided. The guide is positioned relative to the tibia and the talus. The jig includes a body, a fixator for securing the tibia to the body, and an attachment for attaching the guide to the body.

Said jig can be adapted for removably fixedly positioning said guide to said jig, and wherein said jig can be adapted for removably fixedly positioning a second guide to said j ig, said second guide can be adapted for guiding the instrument for the removal of bone from the talus and the tibia of a patient for performing total ankle arthroplasty.

According to a further embodiment of the present invention, a method for providing ankle fusion surgery is provided. The method includes the steps of fixedly securing a talus with respect to a tibia, providing an instrument for removing bone from at least one of a talus and a tibia, providing a guide for guiding the instrument, fixedly securing the guide to the tibia, positioning the instrument in the guide, removing bone from at least one of the tibia and the talus, and fusing the tibia to the talus.

According to yet another embodiment of the present invention, a method for providing ankle surgery is provided. The method includes the steps of analyzing the condition of the tibia and the talus and adjacent soft tissue, and providing an instrument for removing bone from at least one of a talus and a tibia. The method also includes the steps of providing a first guide for guiding the instrument for removal of bone for performing an ankle fusion procedure, providing a second guide for guiding the instrument for removal of bone for performing an total ankle arthroplasty procedure, and fixedly securing one of the first guide and the second guide to the tibia depending on the condition of the tibia and the talus and adjacent soft tissue. The method further includes the steps of positioning the instrument in one of the first guide and the second guide depending on the condition of the tibia and the talus and adjacent soft tissue, removing bone from at least one of the tibia and the talus, and performing one of an ankle fusion procedure and a total ankle arthroplasty procedure depending on the condition of the tibia and the talus and adjacent soft tissue.

The technical advantages of the present invention include reducing the danger of over resection of the joint surfaces. For example, according to one aspect of the invention, the ankle fusion guide of the present invention includes two closely aligned cutting guide slots for the tibia and talus cut. A saw blade is restrained within the guiding slots for providing minimal recession of the tibia and talus. Thus, the present invention provides for reducing the likelihood of over-resection of the joint surfaces.

The technical advantages of the present invention also include improved alignment of the tibia and talus resection cuts for ankle fusion. The improved alignment helps to minimize excessive dorsal flexion and planar flexion, which may cause gait problems or pain. For example, according to one aspect of the present invention, the ankle fusion guide includes a pair of spaced-apart parallel slots for guiding a standard saw blade to perform the tibia and talus resections. Further, according to an aspect of the present invention, a series of pin holes are located in the fusion guide block that are used to securely position the fusion guide block for the resection cuts. Thus, the present invention provides for an accurate alignment of the ankle fusion.

Another technical advantage of the present invention is the ability to keep the resection cuts straight. If the resection cuts are not straight or are angled, then reduced contact occurs within the fusion. For example, if the contact is minimumal, the fusing of the tibia to the talus is delayed. For example, according to one aspect of the present invention, the ankle fusion guide includes a pair of spaced apart guiding slots to which a standard saw blade closely fits. The close fitting of the slots to the standard saw blade results in a straight cut. Thus, the present invention provides for straight cuts during resection.

A further technical advantage of the present invention includes the ability to easily perform an ankle fusion if it appears during surgery that a total ankle replacement is not feasible. For example, according to one aspect of the present invention, the ankle fusion guide is compatible with the alignment of a total ankle arthoplasty instrument set. The cutting guide for a total ankle replacement may be replaced with the ankle fusion guide of the present invention. Thus, the present invention provides for an easy method of performing an ankle fusion if it appears during surgery that a total ankle replacement is not feasible.

A further technical advantage of the present invention is reduced toggling and misalignment of the resection of the tibia and talus during ankle fusion. For example, according to one aspect of the present invention, the ankle fusion block is set even with the interior face of the guide so that the cutting slots can be set flush with the joint. With the additional thickness of the guide, the saw blade has very little room to toggle or misalign. Thus, the present invention provides for additional support for the saw blade such that toggling is minimized. Other technical advantages of the present invention will be readily apparent to one skilled in the art from the following figures, descriptions and claims.

For a more complete understanding of the present invention and the advantages thereof, reference is now made to the following description taken in connection with the accompanying drawings, in which:
FIG. 1 is a plan view of the fusion cutting block in accordance with an embodiment of the present invention mounted in an alignment jig shown in position on a patient;
FIG.2 is a perspective view of the ankle fusion cutting block of Figure 1; FIG. 2A is a cross sectional view of FIG. 2 along the line 2A-LA in the directions of the arrows;
FIG.3 is a plan view of a cutting instrument for use with the ankle fusion cutting block of Figure 1;
FIG. 4 is a front plan view of the ankle fusion cutting block of Figure 1;
FIG.5 is a rear plan view of the ankle fusion cutting block of Figure 1;
FIG.6 is an end view of the fusion cutting block of Figure 1 mounted in an alignment jig shown in position on a patient;
FIG.7 is a partial medial lateral view of a patient showing a fused ankle joint;
FIG. 8 is an end view of a total ankle arthroplasty cutting block mounted in the alignment jig of Figure 1 shown in position on a patient;
FIG.9 is a partial plan view of a patient having a total ankle implant;
FIG. 10 is a partial end view of a patient having a total ankle implant;
FIG. 11 is a process flow chart for a first method of performing ankle fusion surgery according to a further embodiment of the present invention; and
FIG.12 is a process flow chart for another method of performing ankle surgery according to yet another embodiment of the present invention.

Embodiments of the present invention and the advantages thereof are best understood by referring to the following descriptions and drawings, wherein like numerals are used for like and corresponding parts of the drawings.

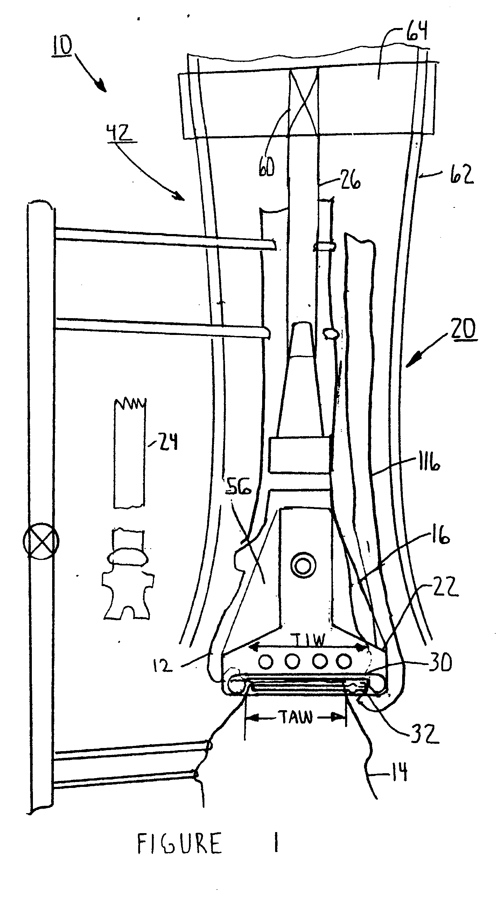

According to the present invention and referring to FIG. 1, a kit 10 for removal of bone 12 from a talus 14 and tibia 16 of a patient 20 is shown. The kit 10 is utilized to perform an ankle fusion procedure. The kit 10 includes a guide 22 for cooperation with at least one of the tibia 16 and talus 14. The kit 10 further includes an instrument 24, which is constrainable, by guide 22 for removal of the bone 12.

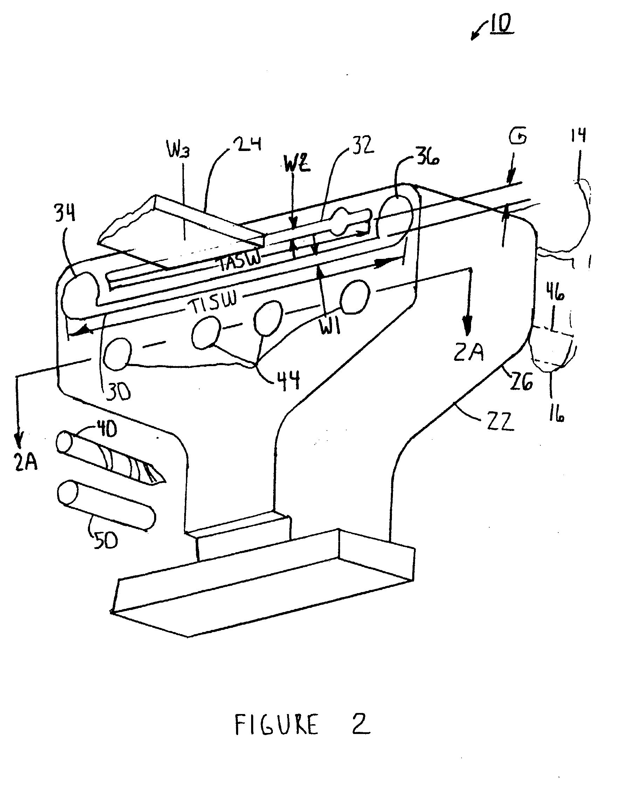

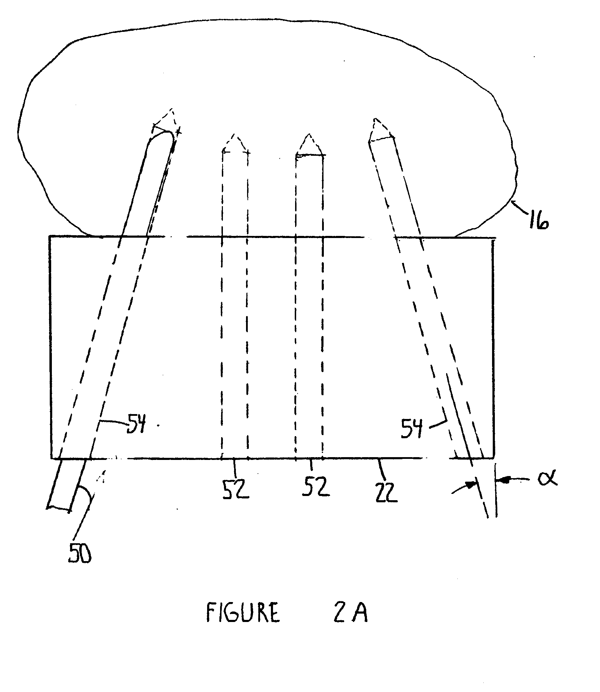

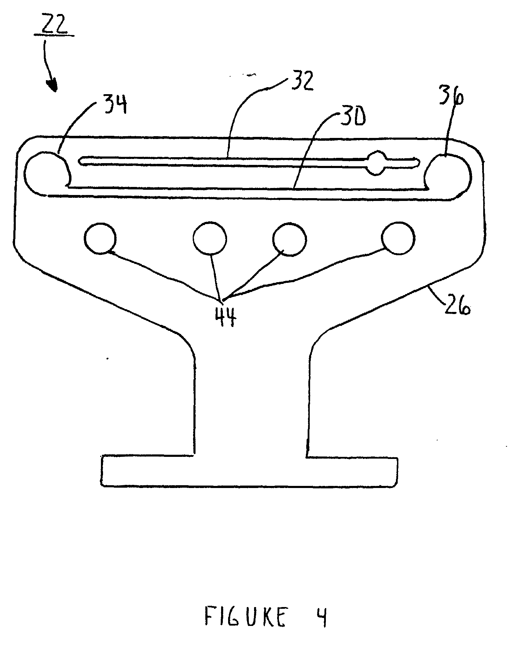

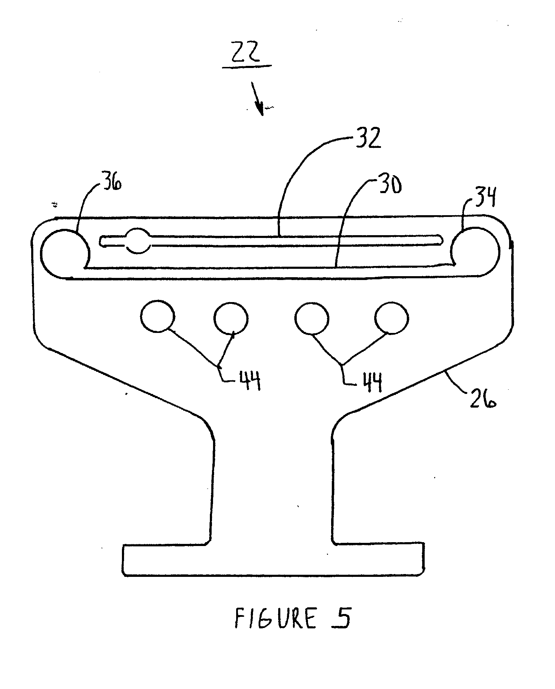

Referring now to FIGS. 2, 4 & 5 the guide 22 of the kit 10 is shown in greater detail. As shown in FIG. 2, the guide 22 may include a body 26. The body 26 may define a first constraint 30 for guiding the instrument 24 for performing the tibia resection. The body 26 may also define a second constraint 32 for guiding the instrument 24 for performing the talus resection. The constraints 30 and 32 may have any suitable shape capable of guiding or controlling the instrument 24. For example, if the instrument 24 is in the form of a cutting blade, the constraints 30 and 32 may be in the form of slots.
To provide for a proper fusion of the ankle, the first constraint or slot 30 and the second constraint or slot 32 are preferably parallel to each other and spaced from each other a distance G. The dimension of the distance G may be slightly larger than the amount the ankle is distracted during the resection. The ankle may be distracted, for example, a distance of approximately one centimeter. The dimension G, thus, may be, for example, approximately 0.1 to 2.5 centimeters. The first slot 30 and the second slot 32 preferably have a width W1 and W2, respectively. The width is selected to permit the instrument 24 to slidably fit within the first and second slots 30 and 32. For example, the instrument 24 may have a width W3 slightly smaller than the widths W1 and W2 of the slots 30 and 32. Referring to FIGS. 1 & 2, in order that the tibia is properly resected, the first slot 30 has a width TISW which is slightly larger than the width TIW of the resected portion of the tibia. Similarly the second constraint 32 has a width TASW which is slightly longer than the width TAW of the resected portion of the talus 14.

Referring again to FIG. 2, the guide 22 may include additional features to guide additional instruments and to provide for particular configuration in the resection. For example, referring to FIG.2, the first slot 30 may include a first relief hole 34 on one end of the first slot 30 and a second relief hole 36 opposed to the first relief hole 34. The relief holes 34 and 36 serve as bushings to guide second instrument 40 in the form of, for example, a drill to prepare arcuate corners in the tibia resections. The relief holes 34 and 36 also serve as a starting point for wire EDM (electronic discharge machining) equipment.

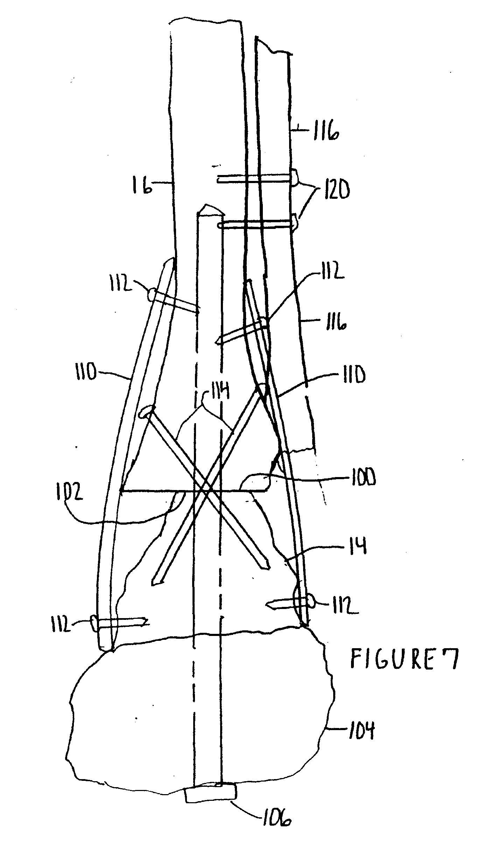

The relief holes 34 and 36 are used to drill a hole through tibia 16 on the medial side of the tibia 16 with drill 40. Referring to FIG. 7, the drill 40 forms arcuate surface 38 between resected surface 100 of tibia 16 and distal medial protrusions 39 of tibia 16. The arcuate surface 38 serves to prevent stress risers in the comers of the resected tibial bone. Further, the relief holes 34 and 36 eliminate the need to run the instrument 24 all the way into the comer of the resected tibia 16.

The first starting hole 34 and 36 are preferably matingly fitted to the drill 40. It should be appreciated that the slots 30 and 32 may not necessarily require the use of relief holes. For example, the second slot 32 does not include a relief hole but merely, as shown in FIG. 2, includes a smaller hole used in manufacturing to assist in the manufacture of the guide 22 by providing a starting point for wire EDM (electronic discharging machining) equipment.

The guide 22 may be made of any suitable durable material capable of constraining the instrument 24. For example, the guide 22 may be made of a metal or ceramic. For example, the guide 22 may be made of a titanium or cobalt chrome alloy or stainless steel. Preferably, the guide 22 is made of a material that is compatible with the human body.

Referring again to FIG. 1, the guide 22, according to the present invention, may be positioned adjacent to the tibia 16 and talus 14 by any suitable method. For example, the guide 22 may be fixably secured to a jig 42. It should be appreciated that the guide 22 may be indirectly secured to the tibia 16 or the talus 14 or secured in any other indirect or direct manner. Preferably, however, to assure accurate and secure positioning guide 22 during the resection of the talus 14 and tibia 16, as shown in FIG.2, the guide 22 may further include mounting holes 44. The mounting holes 44 are for use as bushings for a cutting tool in the form of, for example, the drill 40. The drill 40 is utilized to prepare mounting holes in the tibia 16. The holes 44 and the mounting holes 46 in the tibia are connected utilizing a connector in the form, for example, pins 50.

Referring now to FIG. 2A, the holes 44 are in the form of two parallel spaced-apart inner holes 52 as well as two outer holes 54. As shown in FIG. 2A, the outer holes are oriented at an angle α toward each other. The angle α may be, for example, around 15 degrees. The angling of the outer holes serve to wedge the guide 22 against the tibia 16. As shown in FIG. 2A, preferably, the guide 22 is positioned adjacent to tibia 16 to optimized the accuracy of the resection of the tibias and talus.

Instrument 24 may be, as shown in FIG. 1, in the form of a cutting blade. It should be appreciated the instrument may be in the form of, for example a cutting tool, an osteotome, or a saw. It should be appreciated that preferably the instrument is closely fitted to the constraint or slot in the guide 22.

Referring again to FIG. 1, the jig 42 is shown in greater detail. The jig 42 may include, for example a base or body 26. The guide 22 is connected to the body 26. The body 26 may include the adjustable mounting surface 56 for adjustably mounting the guide 22 to the body 26. The adjustable mounting surface 56 is utilized to properly position the slots 30 and 32 with respect to the tibia 16 and the talus 14.

The body 26, as shown in FIG. 1, may be supported, for example, by means of a mounting arm 60. The mounting arm 60 secures the body 26 to, for example, leg 62 of the patient. The clamp 64 on the mounting arm 62 may be adapted to secure the mounting arm 60 to the leg 62.

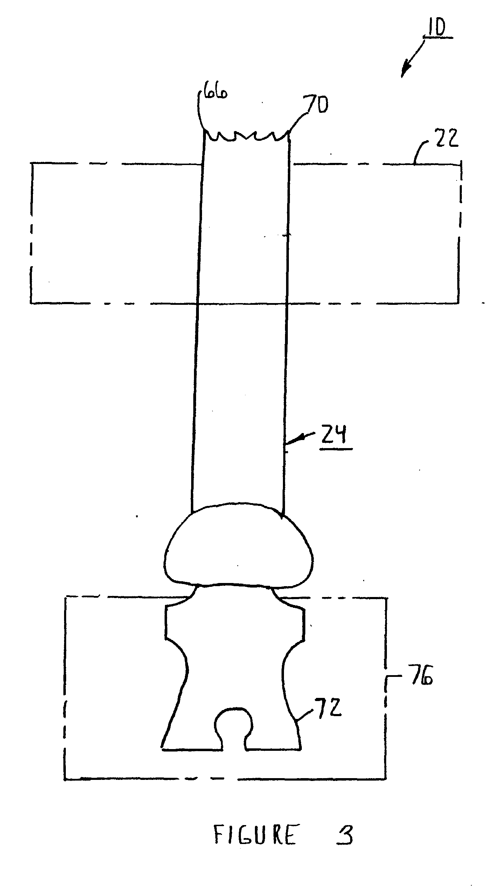

Referring now to FIG. 3, the instrument 24 is shown in greater detail. The instrument 24 may be in the form of a blade and may have teeth 66 located on an end 70 of the instrument 24. The teeth 66 may have any shape suitable for an oscillating blade and may be, for example, the teeth 66 may have teeth such as that of a standard blade from Synvasive Corporation. The instrument 24 may include a connecting end 72 having features for connecting, for example, an oscillating motor 76. The motor 76 may be any standard oscillating motor.

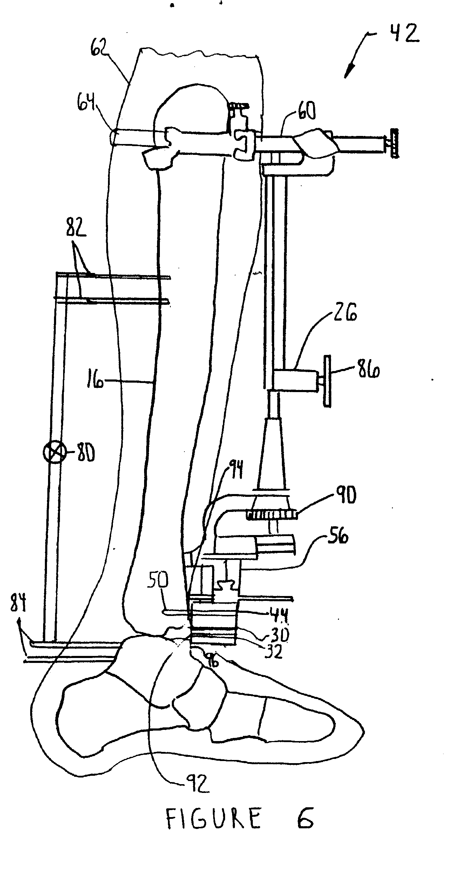

Referring now to FIG. 6, the jig 42 is shown in position on leg 62 of the patient. As shown in FIG. 6 during an operation performing the ankle fusion utilizing the guide of the present invention, an incision is made in the skin between the tibia 16 and the talus 14.

As shown in FIG. 6, a standard ankle distractor 80 is available, for example, commercially available from Orthofix International N.V., Huntersville, North Carolina, is used to distract the ankle joint. The ankle distractor is, for example, secured to the patient by tibia pins 82 which are placed in the tibia 16 and talus distraction pins 84 which are placed in the patient's talus 14 and calcaneus 104. The ankle distractor 80 is actuated to separate the tibia 16 from the talus 14 a distance of, for example, one centimeter.

As shown in FIG. 6, the body 26 of the jig 42 may include a rough adjustment mechanism 86 which permits the guide 22 to the raised and lowered vertically to roughly position the guide 22 in a location between the tibia 16 and the talus 14.

As shown in FIG. 6, the jig 42 may also include a fine tuning adjustment mechanism 90 which provides for precise adjustment of the guide 22 so that it may be precisely positioned between the tibia 16 and talus 14.

Preferably, as shown in FIG.6, the guide 22 may further include a posterior face 92 which preferably is positioned adjacent the interior face 94 of the tibia 16 and adjacent the interior face 96 of the talus 14.

When the guide 22 is properly positioned relative to the tibia 14 and the talus 16, pins 50 are positioned in the holes 44 of the guide 22 to securely hold the guide 22 in position for the resection.

Referring now to FIG. 7, a fused ankle joint is shown utilizing the guide block of the present invention. As shown in FIG. 7, the resected surface 100 of the talus 14 is placed against resected surface 102 of the tibia 16.

Preferably, as shown in FIG. 7, only a minimal amount of bone is removed from the talus 14 and the tibia 16 such as that when the resected surface of 102 are fused together, the leg length of the patient will only be slightly shortened. In such a procedure where the leg length is only slightly shortened (for example, less than one centimeter, the difference in leg length will not be noticed by the patient.

If, due to damage to the talus 14 and/or the tibia 16, it is necessary to remove more material from the talus 14 and the tibia 16, either a bone graft (not shown) may be positioned between the talus 14 and the tibia 16 or a lift or a corrective shoe (not shown) may need to be utilized by the patient to compensate for the difference in the patient's leg length.

Typically, during the ankle fusion, at least the tibia 16 and the talus 14 as well as calcancus 104 are all fused together. The fusing of the talus 14, tibia 16, and calcancus 104 may be accomplished by one of several techniques. For example, an intermedullary rod 106 may be utilized to secure the calcancus 104,talus 14 and tibia 16. Alternatively, the tibia, and talus can be fused together utilizing a combination of bone plates 110 secured to the bones with bone plate screws 112, as well as by using the bone screws 114 to connect the talus to the tibia. During the ankle fusion procedure fibula 116 may, for example, be resected and secured by bone screws 120 to the tibia 16.

From the examination of a patient, including the reading of x-rays, an orthopedic surgeon may determine that a patient is a candidate for a total ankle arthoplasty. Unfortunately, when the patient is brought into the surgery room and an incision is made into the patient around the ankle joint to initiate the total ankle arthoplasty, it may be discovered that due to the condition of the patient's tibia, talus, or adjacent soft tissue, a total ankle arthoplasty is not well advised and an ankle fusion may be the preferred procedure for the patient.

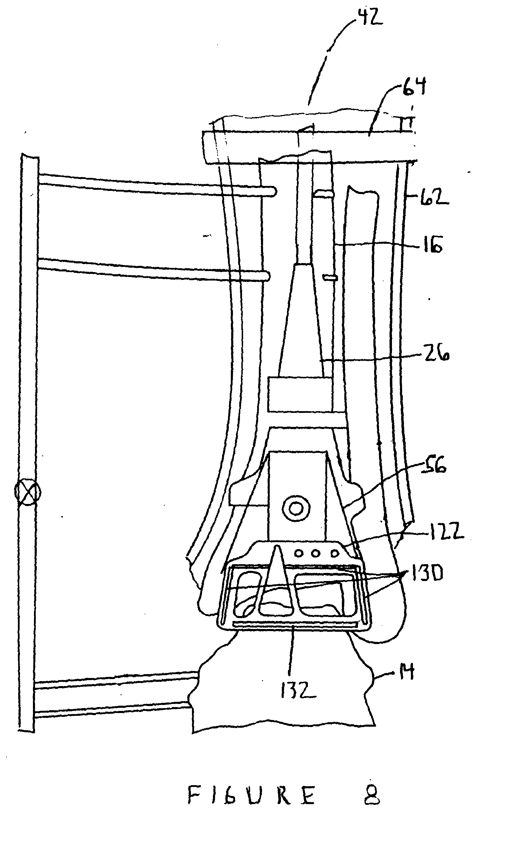

Thus, according to the present invention and referring to FIG.8, the jig 42 may be utilized for either a total ankle arthoplasty or an ankle fusion. For a patient in which the total ankle arthoplasty is planned, the jig 42 may be utilized to support a second guide 122 which is used to prepare the talus 14 and the tibia 16 for the resection cuts necessary to implant a total ankle implant. The second guide 122 is similar to the first guide 22 and may include tibia slots 130 for performing the cuts on the tibia, as well as talus slot 132 for performing the resection of the talus 14. The second guide 122 may be secured, for example, to adjustable mounting surface 56. The second guide 122 may be made of any suitable durable material, for example, a metal, or steel.

As shown in FIG. 8 the jig 42 including the second guide 22 is mounted with clamp 64 around leg 62 of the patient. An insection is made in the patient and the tibia 16 and talus 14, as well as the adjacent soft tissue, is examined to determine whether or not a total ankle arthoplasty is advised for the patient. If the patient is a suitable candidate for the total ankle arthroplasty, the second guide 122 is mounted onto the jig 42. Conversely, if a total ankle arthoplasty is not well advised for the patient, and an ankle fusion is more suitable for the patient, the guide 22 is utilized and positioned in the jig 42.

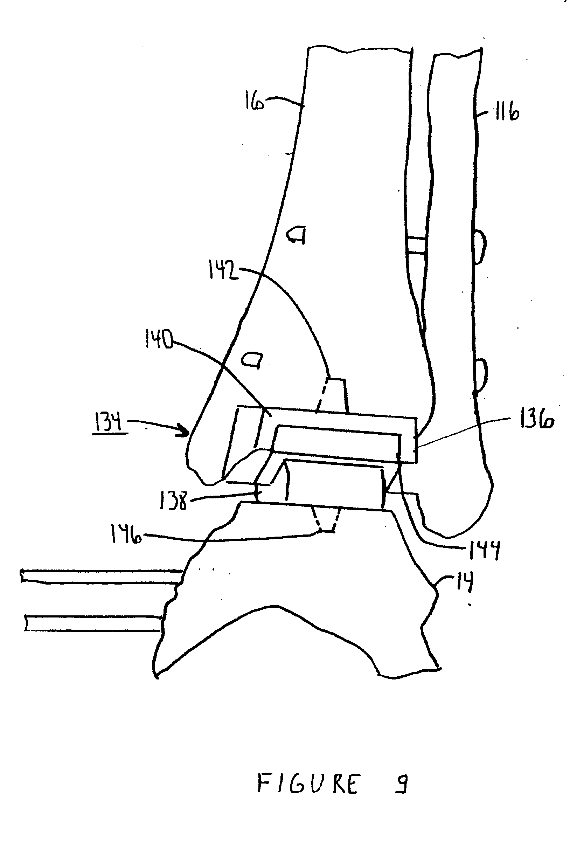

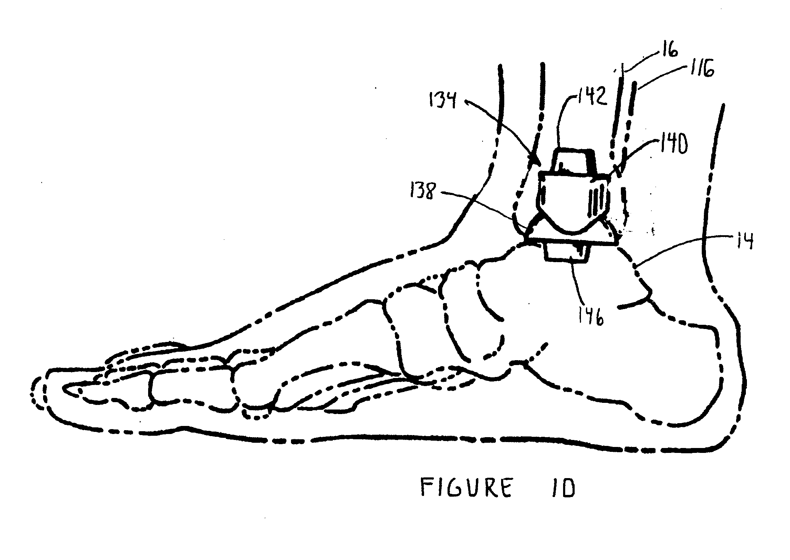

Referring now to FIGS. 9 and 10, a total ankle joint prosthesis or ankle implant 134 is shown implanted into the tibia 16, fibula 116 and talus 14. As shown in FIGS. 9 and 10, the ankle implant 134 is implanted with talar member 138 attaching to the talus 14 and tibia member 136 attaching to the tibia 16 and fibula 116 with base plate 140 bridging the bones. Tibia strut 142 extends up into the tibia 16 when implanted and also positions the tibia member 136 correctly. Bearing 144, typically made of polyethylene, is positioned between tibial member 136 and a talar member 138.

In a similar manner, talar strut 146 extends down into the talus 14 to keep the talar member 138 properly aligned when implanted. The ankle implant 134 is preferably attached without cement as the surfaces of the ankle contacting the bones has a special coating, typically a porous coating to provide for bone in-growth, applied using a surface treatment, for example, microbeads of metal sintered onto the surface.

Since patients have different ankle sizes, different size ankle implants 134 may be required. The correct size is obtained by comparing x-rays of each patient's ankles to an outline of each size implant. It has been found that small, medium, and large size implants provide sufficient variations to accommodate virtually all replacement situations.

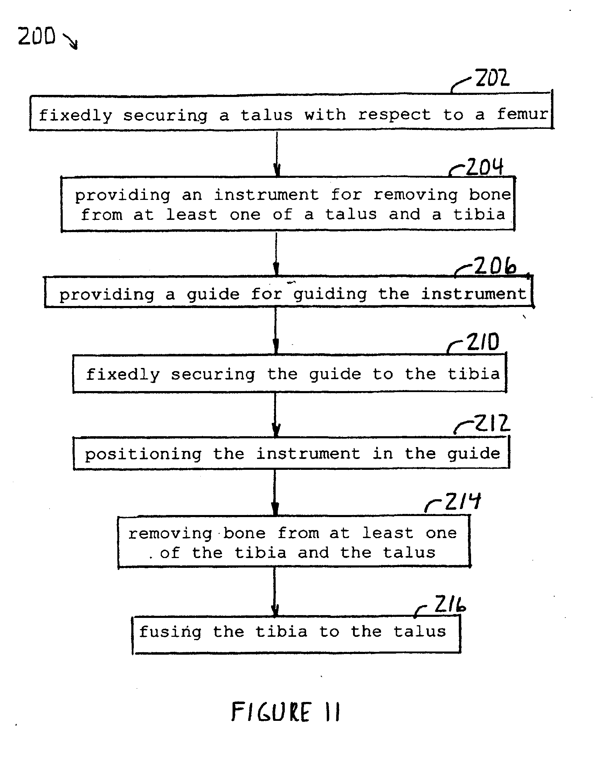

Referring now to FIG. 11, method 200 for providing ankle fusion surgery is described. The method 200 includes a first step 202 of providing an instrument for removing bone from at least one of a talus and a tibia. The method 200 further includes a second step 204 of providing a guide for guiding the instrument.

The method 200 also includes a third step 206 of fixedly securing the guide to the tibia. The method 200 also includes a fourth step 210 of positioning the instrument in the guide. The method 200 also includes a fifth step 212 of removing bone from at least one of the tibia and the talus. The method 200 also includes a sixth step 214 of fusing the tibia to the talus.

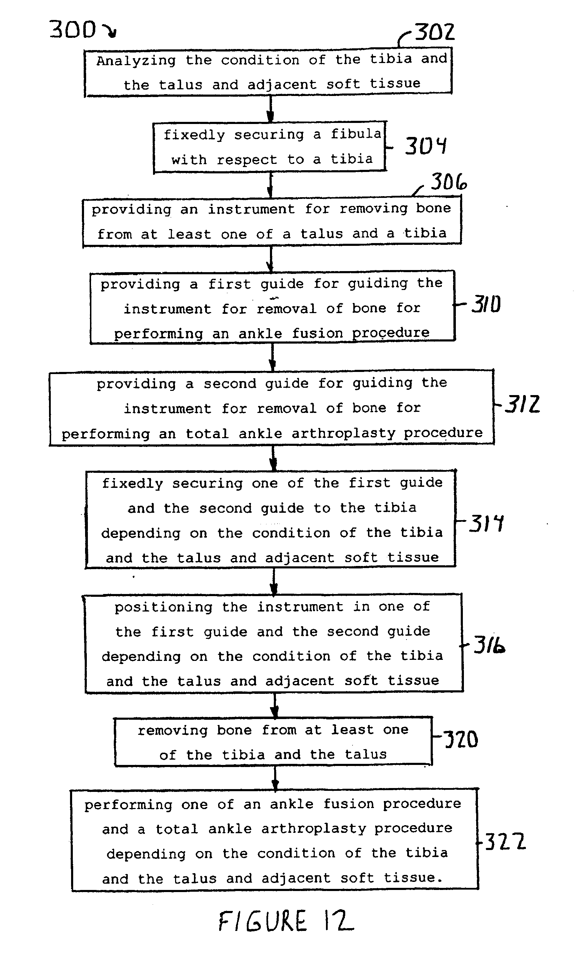

Referring now to FIG. 12, a method 300 for providing ankle surgery includes the first step 302 of analyzing the condition of the tibia and talus and adjacent soft tissue. The method 300 also includes a second step 306 of providing an instrument for removing bone from at least one of the talus and the tibia. The method 300 also includes a third step 310 of providing a first guide for guiding the instrument of removal of bone for performing an ankle fusion procedure.

The method 300 further includes a fourth step 312 of providing a second guide for guiding the instrument for removal of bone for performing a total ankle arthoplasty procedure. The method 300 further includes a fifth step 314 of fixedly securing one of the first guide and the second guide to the tibia depending on the condition of the tibia, the talus and the adjacent soft tissue.

The method 300 further includes a sixth step 316 of positioning the instrument in one of the first guide and the second guide depending on the condition of the tibia, the talus, and the adjacent soft tissue. The method 300 further includes an seventh step 320 of removing bone from at least one of the tibia and the talus. The method 300 also includes a eighth step 322 of performing one of an ankle fusion procedure and a total ankle arthoplasty procedure depending on the condition of the tibia, the talus, and adjacent soft tissue.

The ankle fusion guide, jig, the method of the present invention, provides for an ankle fusion procedure in which a clean even cutting surface for ankle fusions is provided. The use of the guide with its stabilization pins provides a more accurate cut to perform ankle fusion. The ankle fusion guide that is positioned adjacent to the tibia and fibula can provide a guide surface flush with the joint to provide very little room for the saw blade to toggle or misalign during the resection of the tibia and talus.

The jig and method including the use of both a total ankle arthoplasty resection guide, as well as fusion resection guide, provides a surgeon with the comfort of using instruments that may easily perform an ankle fusion if it appears during surgery that total ankle replacement surgery is not feasible.

Although the present invention and its advantages have been described in detail, it should be understood that various changes, substitutions, and alterations can be made therein without departing from present invention as defined by the appended claims.

## Claims

1. A kit for removal of bone from a talus and a tibia of a patient for performing ankle fusion, said kit comprising:
a guide for cooperation with at least one of the tibia and the talus; and
an instrument constrainable by said guide for removal of the bone.

2. The kit of claim 1, wherein said guide comprises a body defining a first constraint for guiding said instrument for performing the tibia resection and defining a second constraint for guiding said instrument for performing the talus resection.

3. The kit of claim 2, wherein at least one of the first constraint and the second constraint is defined by an opening in the body.

4. The kit of claim 3, wherein the opening is a slot.

5. The kit of claim 1, wherein said guide further defines a hole therethrough.

6. The kit of claim 1, wherein said instrument comprises one of a burr tool, an osteotome, a saw and a cutting blade.

7. The kit of claim 1, further comprising a jig for fixedly positioning said guide relative to the tibia and the talus.

8. The kit of claim 7, further comprising a second guide for use in total ankle arthroplasty, said first mentioned guide and said second mentioned guide being removably fixedly postionable to said jig.

9. The kit of claim 1, wherein said guide defines a first through slot, a second through slot spaced from and parallel to the first slot, and a plurality of circular though holes.

10. A guide for guiding an instrument for the removal of bone from a first bone and a second bone of a patient for performing joint fusion therebetween, said guide comprising:
a body; and
a feature operably associated with said body for constraining the instrument.
